(19) Europäisches Patentamt
European Patent Office
Office européen des brevets

(11) Publication number: **0 178 904**
**A2**

(12) # EUROPEAN PATENT APPLICATION

(21) Application number: 85307419.3

(22) Date of filing: 15.10.85

(51) Int. Cl.[4]: C 07 C 46/00
C 07 C 50/34, C 07 C 50/32
C 07 C 50/36

(30) Priority: 19.10.84 US 662683

(43) Date of publication of application:
23.04.86 Bulletin 86/17

(84) Designated Contracting States:
AT BE CH DE FR GB IT LI LU NL SE

(71) Applicant: ENERGY CONVERSION DEVICES, INC.
1675 West Maple Road
Troy Michigan 48084(US)

(72) Inventor: Yu, Terry Ta-Jen
19832 White Oaks Drive
Mt. Clemens Michigan 48083(US)

(72) Inventor: Yen, Mei-Rong
1942 Connolly Drive
Troy Michigan 48098(US)

(74) Representative: Perry, Robert Edward et al,
GILL JENNINGS & EVERY 53-64 Chancery Lane
London WC2A 1HN(GB)

(54) **Quinone synthesis.**

(57) A process for producing a quinone of the formula

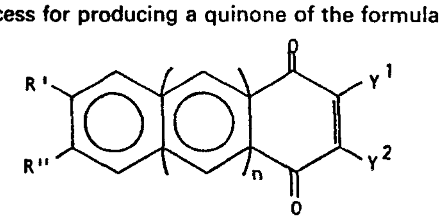

wherein n is zero, 1 or 2; $Y^1$ is alkoxy; $Y^2$ is H, C1, Br or alkoxy; and R' and R" are independently selected from H, alkyl and phenyl, which comprises the steps of:

(a) reacting a tetrabromide of the formula

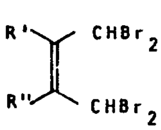 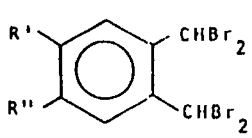 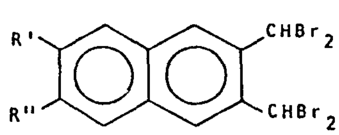

with potassium iodide;

(b) reacting the product of step (a) with a 2-alkoxy-1,4-benzoquinone, to give the desired quinone when $Y^2$ is H;

(c) if desired, reacting the quinone in which $Y^2$ is H with $C1_2$ or $Br_2$, to give the desired quinone in which $Y^2$ is C1 or Br; and

(d) if desired, reacting the quinone in which $Y^2$ is C1 or Br with a lithium, sodium or potassium alkoxide, to give the desired quinone in which $Y^2$ is alkoxy.

EP 0 178 904 A2

QUINONE SYNTHESIS

This invention relates to a process for producing quinones.

US-A-4460678 discloses imaging compositions which comprise a tellurium compound. Quinones are used as reductant precursors, e.g. of the formula

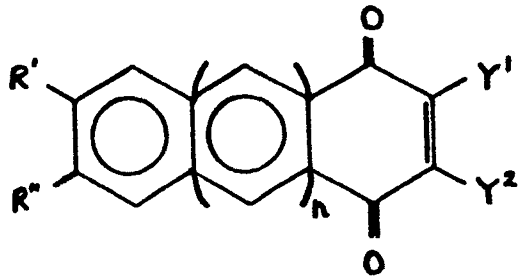

wherein $Y^1$ is $C_{1-5}$ alkoxy, $Y^2$ is H, Cl or $C_{1-5}$ alkoxy, and either n is zero and R' and R" are each H or each phenyl, or n is one and R' and R" are each H. $Y^1$ is $C_{2-5}$ alkoxy, for best results in tellurium imaging film.

Various tellurium compounds useful in imaging proceses, and imaging compositions, both known and new, are disclosed in the other European Patent Application filed in our name on 15th October 1985 (Representative's Reference 70/2527/02; US priority Application No. 662,844).

According to the present invention, a process for producing a quinone of the formula given above, wherein n is zero, 1 or 2; $Y^1$ is alkoxy; $Y^2$ is H; and R' and R" are independently selected from H, alkyl and phenyl, comprises reacting a tetrabromide of the formula

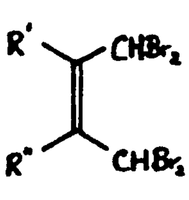

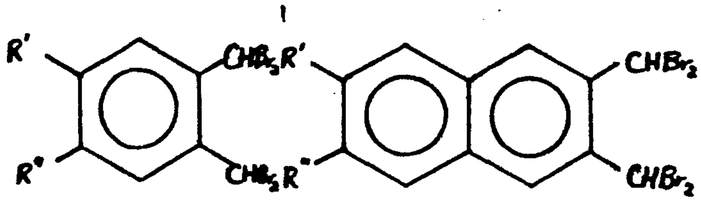

with potassium iodide and a 2-alkoxy-1,4-benzoquinone.

The quinone in which $Y^2$ is H may be reacted with $Cl_2$ or $Br_2$, to give the corresponding quinone in which $Y^2$ is Cl or Br. The quinone in which $Y^2$ is Cl or Br may be reacted with a lithium, sodium or potassium alkoxide, to give the corresponding quinone in which $Y^2$ is alkoxy.

$Y^1$ and $Y^2$ (if alkoxy) usually have 1 to 5 carbon atoms. Examples of each are methoxy, ethoxy, propoxy, butoxy and pentoxy.

The 2-alkoxy-1,4-benzoquinone used as a starting material may be prepared by reacting a 2-alkoxyhydroquinone with an oxidising agent such as lead tetraacetate or lead dioxide. Alkoxyhydroquinones and preparative methods are well known; for example, aniline is oxidised to form quinone, using manganese dioxide, quinone is reduced to form hydroquinone, and hydroquinone is substituted at the 2-position by reaction with sodium alkoxide.

The tetrabromide is usually reacted with potassium iodide prior to reaction with the 2-alkoxy-1,4-benzoquinone. Generally, a better yield is obtained if the 2-alkoxy-1,4-benzoquinone is not added until the potassium iodide is dissolved in the solvent, although the three reactants may be added concurrently. The tetrabromides, and preparative methods, are known.

Without wishing to be bound by theory, it is believed that reaction of the tetrabromide with potassium iodide, with heating, causes the $Br_2C-C=C-CBr_2$ chain to be converted to C=C-C=C, and that the resultant intermediate reacts with the 2-alkoxyhydroquinone.

Representative quinone compounds which can be synthesised in accordance with the process of the invention are 3-chloro-2-isopropoxy-1,4-naphthoquinone; 3-chloro-2-isopropoxy-6,7-diphenyl-1,4-naphthoquinone; 3-chloro-2-isopropoxy-1,4-anthraquinone; 3-chloro-2-(3-pentoxy)-1,4-naphthoquinone; 3-chloro-2-(2-butoxy)-1,4-naphthoquinone; 3-chloro-2-(3,3-dimethyl-2-butoxy)-1,4-naphthoquinone; 2,3-diisopropoxy-1,4-naphthoquinone; 3-chloro-2-methoxy-1,4-naphthoquinone; 2,3-dimethoxy-1,4-naphthoquinone; 3-chloro-2-(t-butoxy)-1,4-naphthoquinone; 3-chloro-2-ethoxy-1,4-naphthoquinone; 3-chloro-2-(n-butoxy)-1,4-naphthoquinone; 3-chloro-2-(2-methylpropoxy)-1,4-naphthoquinone; 2-isopropoxy-1,4-anthraquinone; 2,3-diisopropoxy-1,4-anthraquinone; 3-chloro-2-(2-butoxy)-1,4-anthraquinone; 2,3-dimethoxy-1,4-anthraquinone; 3-chloro-2-(t-butoxy)-1,4-anthraquinone; 3-chloro-2-ethoxy-1,4-anthraquinone and 3-chloro-2-(n-butoxy)-1,4-anthraquinone.

The synthesis method of the present invention is preferably used to make anthraquinones ($n = 1$).

The quinones produced in the invention can be incorporated into organo-tellurium imaging systems as reductant precursors. Their use can result in imaging film having increased sensitivity to visible light at relatively high speed, compared to the use of other reductant precursors.

The synthesis method in accordance with the present invention results in high yields of the quinone. For example, significantly higher yields, of the order of about five to ten times, of the anthraquinone compounds can be obtained, compared to the synthesis methods disclosed in US-A-4460678.

The following Preparation and Examples illustrate the invention. All the reactions were conducted under red light.

<u>Preparation</u>

30 g lead tetraacetate, pre-washed with acetic acid, were added to a suspension of 10 g 2-methoxyhydroquinone in 200 ml benzene. The reaction mixture was stirred at room temperature for 2-3 hours. White lead acetate,

$Pb(OAc)_2$, was formed as a by-product during the reaction, and was removed by filtration. After removal of solvent under reduced pressure, 10 g of crude 2-methoxy-1,4-benzoquinone were obtained.

Example 1

16 g 1,2-bis(dibromomethyl)benzene, 44 g potassium iodide and 10 g crude 2-methoxy-1,4-benzoquinone (product of the Preparation) were stirred in 160 ml N,N-dimethylformamide at 70 C overnight. After the reaction was complete, no bromide was left, as determined by thin layer chromatography, and the reaction mixture was poured into 1200 ml $H_2O$. 10% aqueous $Na_2S_2O_5$ solution was added to decolourise the solution. The precipitate formed in the aqueous solution was collected and dried. Continuous extraction of the dark precipitate, using benzene as a solvent, yielded a dark-coloured solution. The solution was then washed with 2.5% NaOH and $H_2O$, dried and concentrated. 7 g of crude product were obtained. Recrystallisation from methanol yielded 5 g of a pure yellow powder which was 2-methoxy-1,4-anthraquinone (MAQ).

Example 2

4 g chlorine were bubbled into 100 ml carbon tetrachloride. The chlorine solution was then added to a suspension of 4 g MAQ in 100 ml $CCl_4$. After the addition, the reaction mixture was refluxed until it became a clear solution (approximately 4-5 hours). Removal of the solvent yielded a crude oil. 4 g of pure yellow crystals of 2-chloro-3-methoxy-1,4-anthraquinone (CMAQ) were obtained, after recrystallisation from methanol.

Example 3

1 g sodium and 200 ml isopropanol were refluxed to prepare sodium isopropoxide. The alkoxide solution was then added dropwise to a previously cooled suspension of

3 g CMAQ in 100 ml isopropanol, at 0-5 C. The reaction mixture was brought to room temperature and stirred for 30 minutes. After completion of the reaction, 300 ml benzene were added to the mixture. The mixture was then washed with 6 N HCl (to neutralise the mixture), 2.5% NaOH (to remove impurities) and water, dried and concentrated. Recrystallisation of the crude concentrate in isopropanol yielded 2.5 g of pure yellow crystals of 2,3-diisopropoxy-1,4-anthraquinone (DIPAQ).

Example 4

17 ml cold borontrifluoride etherate were added to a suspension of 7 g 2-methoxy-1,4-anthraquinone in 120 ml isopropanol. The reaction was carried out at 70-80 C overnight. After being partially concentrated, the reaction mixture was left at room temperature, and the crude product was obtained as dark brown precipitate. Recrystallisation from isopropanol yielded 5 g of pure yellow needles of 2-isopropyl-1,4-anthraquinone (IPAQ).

Example 5

1 g chlorine was bubbled into a solution of 1 g IPAQ in 50 ml methylene chloride at room temperature. The reaction was complete within 30 minutes. Removal of the solvent yielded a gum-like crude product. After recrystallisation from isopropanol, 1.2 g of pure yellow crystals of 2-chloro-3-isopropoxy-1,4-anthraquinone were obtained.

CLAIMS

1. A process for producing a quinone of the formula

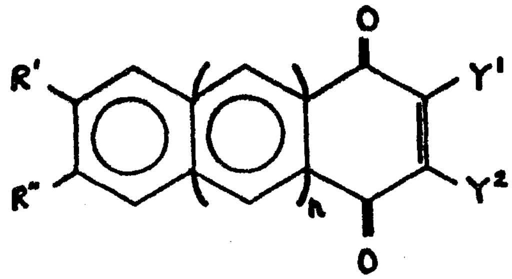

wherein n is zero, 1 or 2; $Y^1$ is alkoxy; $Y^2$ is H, Cl, Br or alkoxy; and R' and R" are independently selected from H, alkyl and phenyl, which comprises the steps of:

(a) reacting a tetrabromide of the formula

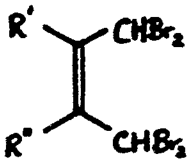

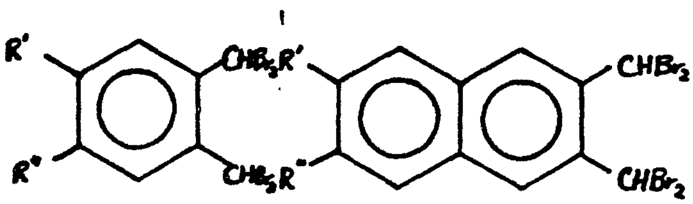

with potassium iodide;

(b) reacting the product of step (a) with a 2-alkoxy-1,4-benzoquinone, to give the desired quinone when $Y^2$ is H;

(c) if desired, reacting the quinone in which $Y^2$ is H with $Cl_2$ or $Br_2$, to give the desired quinone in which $Y^2$ is Cl or Br; and

(d) if desired, reacting the quinone in which $Y^2$ is Cl or Br with a lithium, sodium or potassium alkoxide, to give the desired quinone in which $Y^2$ is alkoxy.

2. A process according to claim 1, in which n is 1 and m is 0, and the product is an anthraquinone.

3. A process according to claim 1, in which n is 0 and the product is a naphthoquinone.

4. A process according to claim 1, in which n is 2 and m is 1.

5. A process according to any preceding claim, in which $Y^1$ and $Y^2$ are each $C_{1-5}$ alkoxy.

6. A process according to claim 5, in which $Y^1$ and $Y^2$ are each isopropoxy.

7. A process according to any of claims 1 to 4, in which $Y^1$ is isopropoxy and $Y^2$ is Cl.